# EUROPEAN PATENT APPLICATION

(11) **EP 4 636 771 A1**
(43) Date of publication of application: **22.10.2025**
(21) Application number: 23904077.7
(22) Date of filing: 15.12.2023
(51) Int. Cl.: G16B 15/00, G16B 25/00, G16B 45/00, G16B 40/00

(54) **DEVICE AND METHOD FOR DISPLAYING PERFORMANCE COMPARISON RESULT WITH RESPECT TO FLUORESCENCE DATA ANALYSIS ALGORITHM**

(30) Priority: 15.12.2022 KR 20220176281
(71) Applicant: Seegene, Inc., Seoul 05548 (KR)
(72) Inventor: SON, Hyungsuk, Seoul 05548 (KR); KIM, Jihye, Seoul 05548 (KR); KIM, Hyounggyu, Seoul 05548 (KR); PARK, Bong Gyun, Seoul 05548 (KR); LEE, Yeji, Seoul 05548 (KR)
(74) Representative: Viering, Jentschura & Partner mbB Patent- und Rechtsanwälte
(86) International application number: PCT/KR2023/020818
(87) International publication number: WO 2024/128878

(57) **Abstract**

A computer device according to one embodiment may include: a memory that stores at least one instruction, fluorescence data for an amplification reaction of a target analyte, and legacy analysis data for the target analyte, the legacy analysis data being a result of applying a legacy analysis algorithm to the fluorescence data; a display unit; and a processor. The at least one instruction may be executed by the processor to obtain update analysis data for the target analyte, which is a result of applying an update analysis algorithm of the legacy analysis algorithm to the fluorescence data, and to display a comparison result of the legacy analysis data and the update analysis data on the display unit.

## Description

### TECHNICAL FIELD

The present disclosure relates to a device and method for displaying a performance comparison result with respect to a fluorescence data analysis algorithm.

### BACKGROUND ART

Molecular diagnosis is a technique used to identify and analyze nucleic acids or proteins at a molecular level. Detailed techniques of such molecular diagnosis include polymerase chain reaction (PCR), isothermal nucleic acid amplification technology (INAAT), DNA sequencing & next-generation sequencing (NGS), in situ hybridization (ISH), DNA microarray, etc.

In each technique, several steps are performed to identify and analyze target materials. In this case, to analyze the target materials, molecular diagnosis data is analyzed through various analysis algorithms at each step.

Such analysis algorithms may be changed according to the change in the diagnostic environment, such as target materials, analysis reagents, and analysis techniques. The changed analysis algorithm is compared and evaluated with the existing analysis algorithm, thereby evaluating or determining whether to improve the performance of the analysis algorithm or whether to apply the analysis algorithm.

### DETAILED DESCRIPTION OF THE DISCLOSURE

### TECHNICAL PROBLEMS

The present disclosure is directed to providing a technology for displaying a performance comparison result with respect to an analysis algorithm described above, but is not limited thereto.

### MEANS FOR SOLVING PROBLEMS

A computer device according to one embodiment includes: a memory that stores at least one instruction, fluorescence data for an amplification reaction of a target analyte, and legacy analysis data for the target analyte, the legacy analysis data being a result of applying a legacy analysis algorithm to the fluorescence data; a display unit; and a processor, wherein the at least one instruction is executed by the processor to obtain update analysis data for the target analyte, which is a result of applying an update analysis algorithm of the legacy analysis algorithm to the fluorescence data, and to display a comparison result of the legacy analysis data and the update analysis data on the display unit.

Further, the fluorescence data may be a fluorescence signal detected in a reaction vessel where a nucleic acid amplification reaction occurs.

Further, the legacy analysis algorithm and the update analysis algorithm may include at least one of a sub-module used for processing the fluorescence data and a sub-module used for determining the presence or absence of the target analyte in a sample.

Further, the update analysis algorithm may be generated by modifying or excluding one or more of sub-modules in the legacy analysis algorithm, or generated by adding one or more sub-modules other than the sub-modules in the legacy analysis algorithm, or generated by changing an execution sequence of one or more of the sub-modules in the legacy analysis algorithm.

Further, the memory may store a plurality of sets of fluorescence data and legacy analysis data for the plurality of sets of fluorescence data, the at least one instruction may be executed by the processor to input a search condition and then search for the fluorescence data using the search condition among the plurality of sets of fluorescence data, and the obtained update analysis data may be a result of applying the update analysis algorithm to the searched fluorescence data.

Further, the memory may store metadata for each of the plurality of sets of fluorescence data, and the search condition may include metadata.

Further, the metadata may include one or more selected from the group consisting of information generated during a development process of a reagent for detecting the target analyte, information on the reagent, information on the target analyte, identification (ID) information on a sample used in the nucleic acid amplification reaction, identification (ID) information on a reaction vessel used in a reaction to obtain amplification data, temperature information, enzyme master mix information, equipment information, and consumables information.

Further, the plurality of sets of fluorescence data may be obtained in respective reaction vessels and are applied to either the legacy analysis algorithm or the update analysis algorithm.

Further, the reaction vessel may be a well, a well strip, or a plate.

Further, the legacy analysis data may be data which have been prepared to verify performance of a diagnostic reagent of the target analyte for submitting to a predetermined certification authority, and when the comparison result of the legacy analysis data and the update analysis data falls within a predetermined range, an update from the legacy analysis algorithm to the update analysis algorithm may be executed.

Further, the computer device may further include: a data normalization unit, wherein the memory stores the fluorescence data and the legacy analysis data as a plurality of data sets, and the plurality of sets of fluorescence data and legacy analysis data are standardized by the data normalization unit.

Further, the display unit may display a determination comparison result of the presence or absence of the target analyte for the legacy analysis data and the update analysis data, or display a Ct value comparison distribution of the target analyte for the legacy analysis data and the update analysis data.

Further, the legacy analysis data may be prepared to verify performance of a diagnostic reagent of the target analyte for submitting a predetermined certification authority.

According to one embodiment, a method for comparing and analyzing a plurality of algorithms for analyzing fluorescence data for an amplification reaction of a target analyte inlcudes: storing fluorescence data for the target analyte and legacy analysis data for the target analyte, which is a result of applying a legacy analysis algorithm to the fluorescence data; obtaining update analysis data for the target analyte, which is a result of applying an update analysis algorithm of the legacy analysis algorithm to the fluorescence data; and displaying a comparison result of the legacy analysis data and the update analysis data.

According to one embodiment, a method for comparing and analyzing a plurality of algorithms for analyzing amplification data obtained from a nucleic acid amplification reaction of the target analyte, the method being performed by a computer device, include: receiving the amplification data and a legacy analysis algorithm, the amplification data including i) metadata for the nucleic acid amplification reaction and ii) fluorescence data indicating an amplification result of the nucleic acid amplification reaction; generating normalized data using data extracted and converted from the input amplification data; storing i) the normalized data and ii) legacy analysis data which is a result of applying the legacy analysis algorithm to the normalized data in a normalized database (DB) pre-built in a memory of the computer device, the normalized DB storing, for the target analyte, a plurality of normalized data and legacy analysis data for the plurality of normalized data; receiving a search condition including the metadata and/or the fluorescence data; searching for normalized data meeting the search condition among the plurality of normalized data stored in the normalized DB and legacy analysis data for the normalized data; receiving an update analysis algorithm of the legacy analysis algorithm; comparing update analysis data, which is a result of applying the update analysis algorithm to the searched normalized data, with the searched legacy analysis data; and displaying a comparison result.

Further, the metadata may include one or more selected from the group consisting of information generated during a development process of a reagent for detecting the target analyte, information on the reagent, information on the target analyte, identification (ID) information on a sample used in the nucleic acid amplification reaction, identification (ID) information on a reaction vessel used in a reaction to obtain the amplification data, temperature information, enzyme master mix information, equipment information, and consumables information.

Further, the fluorescence data may be a fluorescence signal detected in a reaction vessel where the nucleic acid amplification reaction occurs.

Further, the normalized data may be generated by converting data extracted from the metadata so that the received metadata conforms to a predetermined format.

Further, the metadata included in the normalized data may be structured in a hierarchy representing a parent-child relationship among data.

A computer program according to one embodiment may be stored on a computer-readable recording medium programmed to perform, each step included in the method described above.

A computer-readable recording medium according to one embodiment may be stored a computer program programmed to perform, each step included in the method described above.

### EFFECTS OF INVENTION

According to one implementation example, various effects to be described below can be obtained.

First, it is possible to confirm a performance comparison result between a legacy analysis algorithm for molecular diagnosis data and update analysis algorithm of the legacy analysis algorithm.

Second, it is possible to easily confirm whether the update analysis algorithm has been improved compared to the legacy analysis algorithm. As a result, it is possible to determine whether to apply the update analysis algorithm.

Third, it is possible to easily determine whether the update analysis algorithm has been updated or changed to a level that requires molecular diagnostic certification and/or authorization.

### BRIEF DESCRIPTION OF THE DRAWINGS

FIG. 1 illustrates a detection device and a display device for displaying a performance comparison result with respect to a fluorescence data analysis algorithm connected to the detection device according to one embodiment.
FIG. 2 conceptually illustrates a configuration of the display device according to one implementation example.
FIG. 3 illustrates a type of information stored in a memory.
FIG. 4 conceptually illustrates a configuration in which each result data generated by applying multiple algorithms to fluorescence data is compared with each other according to one implementation example.
FIG. 5 conceptually illustrates a configuration in which an algorithm is updated according to one implementation example.
FIG. 6 conceptually illustrates a configuration in which fluorescence data generates positive and negative determining results of a target analyte through a plurality of sub-modules of a DSP algorithm according to one implementation example.
FIG. 7 illustrates analysis data for a legacy analysis algorithm and an update analysis algorithm, plotted on a Ct - α4 plane, according to one implementation example.
FIG. 8 illustrates Ct values for each of the legacy analysis algorithm and the update analysis algorithm, plotted on one plane, according to one implementation example.
FIG. 9 is a flowchart of a method for displaying an algorithm comparison result according to one implementation example.

### BEST MODE FOR IMPLEMENTING THE INVENTION

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

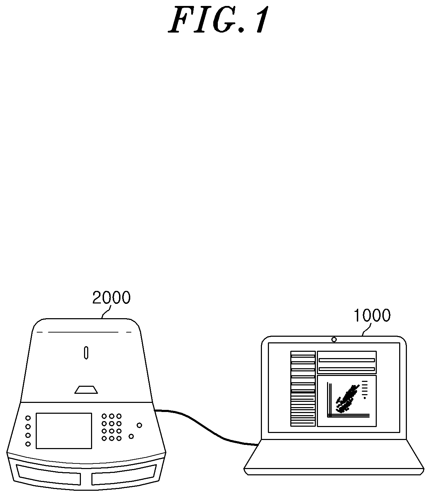

Before explaining FIG. 1, the terms used in the present application will be described.

The term "plate" refers to a standard unit in which an amplification reaction is performed in a PCR device, and means a basic unit in which data generated after the amplification reaction is stored. Different plates may be plates in which an amplification reaction is performed at different times using the same amplification device, or plates in which the amplification reaction is performed at the same time by different amplification devices.

The plate includes a plurality of reaction wells. The plate may include N × M reaction wells. Typically, the plate includes 12 × 8 or 8 × 12 reaction wells. The reaction wells of the plate may be in an integral type with the plate or may be in the form of a separable tube. The plate may be rectangular, but the plate may include one or more reaction wells and may be implemented in various shapes such as a circle, a ladder, and a diamond in addition to the rectangle.

The wells of the plate include a sample to be analyzed and reagents necessary for a nucleic acid amplification reaction.

The term "target analyte" includes various materials (e.g., biological materials and non-biological materials), which may refer to the same subject as the term "target analyte. "

Such a target analyte may specifically include biological materials, and more specifically, include at least one of nucleic acid molecules (e.g., DNA and RNA), proteins, peptides, carbohydrates, lipids, amino acids, biological compounds, hormones, antibodies, antigens, metabolites, or cells.

The term "sample" refers to both biological samples (e.g., cells, tissues, and body fluids) and non-biological samples (e.g., food, water, and soil). Among these, the biological samples may include at least one of viruses, bacteria, tissues, cells, blood (including whole blood, plasma, and serum), lymph, bone marrow fluid, saliva, sputum, swab, aspiration, milk, urine, stool, eye fluid, semen, brain extracts, spinal fluid, synovial fluid, thymic fluid, bronchial lavage fluid, ascites, or amniotic fluid. Such samples may or may not include the above-described target analyte.

Meanwhile, when the target analyte described above is a nucleic acid molecule or includes a nucleic acid molecule, a nucleic acid extraction process known in the art may be performed on the sample estimated as including the target analyte (see: Sambrook, J. et al., Molecular Cloning. A Laboratory Manual, 3rd ed. Cold Spring Harbor Press (2001)). The nucleic acid extraction process may vary depending on a type of samples. In addition, when the extracted nucleic acid is RNA, it may additionally undergo a reverse transcription process to synthesize cDNA (see Sambrook, J. et al., Molecular Cloning. A Laboratory Manual, 3rd ed. Cold Spring Harbor Press (2001).

The term "data set" refers to data obtained from a signal generation reaction for the target analyte using a signal generation means (the signal generation means will be described below).

In this case, the term "signal generation reaction" means a reaction that generates a signal depending on properties of the target analyte in the sample, such as activity, amount, or presence (or absence), specifically presence (or absence). Such a signal generation reaction includes a biological reaction and a chemical reaction. Among these, the biological reaction includes a genetic analysis process such as PCR, real-time PCR, and microarray analysis, an immunological analysis process, and bacterial growth analysis. In addition, the chemical reaction includes a process of analyzing formation, change, or destruction of chemicals. According to an implementation example, the signal generation reaction may be the genetic analysis process, or may be a nucleic acid amplification reaction, an enzyme reaction or microbial growth.

Meanwhile, the signal generation reaction described above is accompanied by a change in signal. Therefore, the progress of the signal generation reaction may be evaluated by measuring the change in the signal.

Here, the term "signal" means a measurable output. In addition, the measured size or change of the signal serves as an indicator that qualitatively or quantitatively indicates the properties of the target analyte, specifically, the presence or absence of the target analyte in the sample.

Here, examples of the indicator include, but are not limited to, fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectivity, transmittance, and absorbance.

The term "signal generation means" as described above means a means for providing the signal indicating the properties of the target analyte to be analyzed, specifically, the presence or absence of the target analyte.

The signal generation means include a label itself or an oligonucleotide to which the label is linked.

Among these, the label includes a fluorescent label, a luminescent label, a chemiluminescent label, an electrochemical label, and a metal label. The label may be used as the label itself, such as an intercalating dye. Alternatively, the label is the form of a single label or an interactive dual label including a donor molecule and an acceptor molecule, and may be used in the form that it is bonded to one or more oligonucleotides.

When using the fluorescent label, a signal value may be expressed as a relative fluorescence unit (RFU) value.

The signal generation means may additionally include an enzyme having nucleic acid cleavage activity to generate a signal (for example, an enzyme having 5' nucleic acid cleavage activity or an enzyme having 3' nucleic acid cleavage activity).

Meanwhile, various methods have been known for generating a signal indicating the presence of the target analyte, particularly the target nucleic acid molecule, using the signal generation means. Representative examples of the methods may include the following: TaqManTM probe method (US Patent No. 5,210,015), molecular beacon method (Tyagi, Nature Biotechnology, v.14 MARCH 1996), Scorpion method (Whitcombe et al., Nature Biotechnology 17:804-807 (1999)), Sunrise (or Amplifluor) method (Nazarenko et al., Nucleic Acids Research, 25(12):2516-2521 (1997), and US Patent No. 6,117,635), Lux method (US Patent No. 7,537,886), CPT (Duck P, et al. Biotechniques, 9:142-148 (1990)), LNA method (US Patent No. 6,977,295), Plexor method (Sherrill CB, et al., Journal of the American Chemical Society, 126:4550-4556 (2004)), Hybeacons (D.J. French, et al., Molecular and Cellular Probes 13:363-374 (2001) and US Patent No. 7,348,141), dual-labeled, self-quenched probe (US Patent No. 5,876,930), hybridization probe (Bernard PS, et al., Clin Chem 2000, 46, 147-148), PTOCE (PTO cleavage and extension) method (WO 2012/096523), PCE-SH (PTO Cleavage and Extension-Dependent Signaling Oligonucleotide Hybridization) method (WO2013/115442), PCE-NH(PTO Cleavage and Extension-Dependent Non-Hybridization) method (PCT/KR2013/012312) and CER method (WO 2011/037306).

Meanwhile, the above-described term "signal generation reaction" may refer to an amplification reaction of a signal. In this case, the term "amplification reaction" means a reaction that increases or decreases a signal generated by the signal generation means. Specifically, the amplification reaction means an increase (or amplification) reaction of a signal generated by the signal generation means depending on the presence of a target analyte.

In this amplification reaction, the amplification of the target analyte (e.g., a nucleic acid molecule) may or may not be accompanied. More specifically, the amplification reaction may mean the amplification reaction of the signal accompanied by the amplification of the target analyte.

Meanwhile, a data set obtained through the amplification reaction may include an amplification cycle. In this case, the data set is referred to as "fluorescence data."

Here, the term "cycle" refers to a unit of change in the condition in multiple measurements accompanying a change in a certain condition. The change in the certain condition means, for example, an increase or decrease in temperature, reaction time, the number of times of reactions, concentration, pH, the number of times of replications of a measurement target (e.g., nucleic acid), etc. Therefore, the cycle may be a time or process cycle, a unit operation cycle, or a reproductive cycle.

More specifically, when a reaction of a certain process is repeated or a reaction is repeated based on a certain time interval, the term "cycle" may mean one unit of the repetition.

For example, in the case of the polymerase chain reaction (PCR), one cycle means a reaction including a denaturation step of a nucleic acid, an annealing step of a primer, and an extension step of a primer. In this case, the change in the certain condition is an increase in the number of times of repetitions of the reaction, and a unit of repetition of the reaction including the series of steps is set as one cycle.

Alternatively, the term "cycle" may mean one unit of the repetition when a certain action is repeated according to the progress of the reaction.

For example, when the nucleic acid amplification reaction is performed, an action of detecting a signal generated at a certain time interval may be repeated, and may mean one unit of the repetition. In this case, the cycle may have a unit of time. Meanwhile, an amplification reaction for amplifying a signal indicating the presence of the target analysis material (or the target analyte) may be performed by a method (e.g., real-time PCR method) in which a signal is also amplified while a target analyte is amplified. Alternatively, according to one implementation example, the amplification reaction may be performed by a method (e.g., CPT method (Duck P, et al., Biotechniques, 9:142-148(1990)), Invader assay (US Pat. Nos. 6,358,691 and 6,194,149)) in which only a signal indicating presence of a target analyte is amplified without amplifying the target analyte.

Meanwhile, the above-described target analysis material or target analyte, particularly the target nucleic acid molecule, may be amplified by the following various methods: polymerase chain reaction (PCR), ligase chain reaction (LCR) (U.S. Pat. Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatchet et al., Genet. Anal. 15(2):35-40 (1999)) and Q-Beta Replicase (Lizardi et al., BiolTechnology 6:1197(1988)), loop-mediated isothermal amplication (LAMP, Y. Mori, H. Kanda and T. Notomi, J. Infect. Chemother., 2013, 19, 404-411), Recombinase polymerase amplification (RPA, J. Li, J. Macdonald and F. von Stetten, Analyst, 2018, 144, 31-67).

Meanwhile, the amplification reaction amplifies the signal while amplifying the target analyte (specifically, the target nucleic acid molecule). For example, the amplification reaction is performed either by the PCR, specifically the real-time PCR, or by the isothermal amplification reaction (e.g., LAMP or RPA).

Meanwhile, the data set obtained by the signal generation reaction includes a plurality of data points including cycles of the signal generation reaction and signal values in the cycles.

Here, the term "signal values" mean numerical values of a level (e.g., signal intensity) of a signal actually measured in the cycle of the signal generation reaction, particularly the amplification reaction based on a certain scale, or modified values of the numerical values. The modified values may include mathematically processed signal values of the actually measured signal values. Examples of the mathematically processed signal values of the actually measured signal values (i.e., signal values of a raw data set) may include logarithmic values or derivatives.

The term "data point" means a coordinate value including the cycle and the signal value. In addition, the term "data" means all information constituting a data set. For example, each of the cycles and the signal values of the amplification reaction may correspond to data.

The data points obtained by the signal generation reaction, particularly the amplification reaction, may be expressed as coordinate values that may be represented in a two-dimensional rectangular coordinate system. In the coordinate values, an X-axis represents the number of corresponding cycles, and a Y-axis represents the signal values measured or processed in the corresponding cycle.

The term "data set" means a set of the data points. For example, the data set may be the set of the data points obtained directly through the amplification reaction performed in the presence of the signal generation means, or may be a transformed data set that has transformed such a data set. The data set may be a part or all of the plurality of data points obtained by the amplification reaction or the modified data points thereof. In addition, the data set may be plotted, so an amplification curve may be obtained.

Meanwhile, the data set may be a data set obtained by processing a plurality of data sets. When analysis of a plurality of target analytes is performed in one reaction vessel, in some cases, the data set for the plurality of target analytes may be obtained by processing the data sets obtained from the reaction performed in the one reaction vessel. For example, the data set for the plurality of target analytes performed in one reaction vessel may be obtained by processing the plurality of data sets obtained from signals measured at different temperatures.

In one embodiment, the reaction vessel provides a closed space in which the reaction is performed. The reaction vessel includes one or two more reaction containers. The reaction container refers to a unit that may accommodate a reactant (e.g., a reaction solution or a reaction mixture). Each of a test tube, a PCR tube, a strip tube, and a multi-well PCR plate is an implementation example of a reaction vessel containing one or more reaction containers.

The term "algorithm" is a series of signal processing processes composed of at least one sub-module. In this specification, "analysis algorithm" means an algorithm to which fluorescence data may be applied.

For example, the analysis algorithm to which the fluorescence data is applied may include DSP and BPN.

The BPN is one of the methods for correcting and analyzing a data set. The BPN may provide a normalization coefficient for correcting a data set obtained from a signal generation reaction for a target analyte using a signal generation means, in which the normalization coefficient being provided using a data set that includes a reference value, a reference cycle, and a plurality of data points including cycles of the signal generation reaction and signal values in the cycles, the cycle is selected from the cycles of the data set, the reference value is an arbitrarily determined value, the normalization coefficient is provided by determining a relationship between the signal value of the cycle corresponding to the reference cycle in the data set and the reference value, and apply the normalization coefficient to the signal values of the data set to obtain the corrected signal values, thereby providing the corrected data set to perform the analysis.

The DSP may obtain a data set for a target analyte from a signal generation reaction using a signal generation means to analyze the target analyte without false positive and false negative results, particularly false positive results, by using fitting accuracy of a non-linear function for the data set as a direct indicator for target analysis of a target analyte, correct the obtained data set including a plurality of data points including cycle numbers and signal values, generate the non-linear function for the corrected data set to determine fitting accuracy of the non-linear function for the corrected data set, and determine presence or absence of the target analyte in the sample using the fitting accuracy. The analysis method requires various parameters, and may be optimized according to values set for each parameter. Using the BPN (WO2017/086762) and DSP (WO2019/066572) technology, parameters used in the process of determining the presence or absence of the target analyte may be assigned, and an interface for setting values of the BPN and DSP parameters may be provided.

These parameters are used for mathematical processing of a signal in a signal generation reaction that generates a signal dependent on the presence of the target analyte, or used as a criterion for determining the presence or absence of the target analyte in the sample. Examples of values that may determine the positive and negative of the sample include Ct (threshold cycle), delta Ct, melt Tm, a melt peak, a threshold intensity value of the signal used for Ct determination, a threshold value for determining the Ct value; a fitting start cycle and final cycle used for background subtraction; a reference value and a reference cycle when adopting an algorithm disclosed in WO 2017/086762; a R2 value, a maximum slope value of a sigmoid fitting curve, and difference between a maximum signal value and a minimum signal value when using a sigmoid fitting algorithm; and a reference value when adopting the algorithm disclosed in WO2015-147370, WO 2015/147412, WO2015/147382, and WO2016/093619.

Therefore, the BPN of the analysis method may include parameters for a reference value (RV) and a signal value relationship parameter (cut-off ratio (CR)) value. The CR numerically describes, in particular, a relationship or degree of a change in signal, a signal change, or a signal difference when two signal values occur at different detection temperatures. In this case, the different detection temperatures may be a relative high detection temperature and a relative low detection temperature, or a first temperature and a second temperature. The "signal value relationship parameter" includes any value that reflects a pattern (rule) of a signal change at different detection temperatures. In addition, the signal value relationship parameter refers to a value representing a degree of change between a signal detected at a relative high detection temperature and a signal detected at a relative low detection temperature for a specific target nucleic acid sequence. The signal value relationship parameter may refer to a value used to convert, switch, adjust, or transform a signal detected at one temperature into a signal detected at another temperature. The signal value relationship parameter may vary depending on the type of the target nucleic acid sequence, the type of the signal generation means, and the conditions of incubation and detection. Therefore, various signal value relationship parameters may be determined for different or identical target nucleic acid sequences.

The signal value relationship parameters may be expressed in various forms. For example, the signal value relationship parameter may be expressed as a numerical value, the presence/absence of the signal, or a plot with signal characteristics.

The DSP includes parameters for correcting a data set obtained through a signal generation reaction and parameters for numerical adjustment. The parameters for correcting the data set may include a parameter criterion (PMC) for determining a shape of a sigmoid graph for a data set obtained through a signal generation reaction, a start fitting cycle (SFC) for correcting a baseline fitting start cycle, and a minimum fitting cycle (MFC) used for baseline fitting from the SFC to the MFC to correct the data set.

The parameters for numerical adjustment may include a value (cut-off ratio (CR)) obtained by dividing an RFU value at low temperature by an RFU value at high temperature in order to compensate for a difference in REU values due to a difference in low/high RFU values, a threshold value (THRD, Ct threshold) for reading positive and negative samples after sigmoid fitting, a determinant RFU (DRFU) for filtering the positive and negative samples, and an R square criterion (RC) for processing negative when R² is less than or equal to the RC. In addition, US Patent Laid-Open Publication No. 2009/0119020 may determine whether the data set shows significant or valid growth by using the R² value of the sigmoid function. In this case, the parameters for numerical adjustment may further include the parameter RC indicates negative when the R² value is less than or equal to the R square criterion These parameters may have values set for each detection channel (FAM, HEX, C610, Q670, and Q705) and detection temperature.

The values for each parameter according to the type of this analysis method are values for determining the positive and negative to converge to the positive and negative determining results confirmed in clinical trials.

The term "sub-module" is a predetermined unit of the signal processing process that analyzes or processes the signal values obtained during the detection process of the target. For example, correcting the data set in the DSP, generating the non-linear function for the corrected data set to determine the fitting accuracy of the non-linear function for the corrected data set, and determining the presence or absence of the target analyte in the sample using the fitting accuracy may each be the sub-module.

As described above, the sub-module may include parameters for performing each signal processing process. Here, the value of the parameter may be a setting value required for the sub-module to operate. For example, the parameters may be a kind of reference values for positive and negative determination, or a kind of parameters required for mathematically modeling the signal, but is not limited thereto. In addition, the parameters may have values or ranges of pre-assigned parameters. In addition, the parameters may have their values or ranges of the pre-assigned parameters updated. Also, the values or ranges of the parameters may not be assigned in advance.

The term "execution sequence" refers to the type of sub-modules that constitute an algorithm and the order in which the sub-modules are arranged.

Meanwhile, the algorithm may be "updated" by changing the type or order of the sub-modules that constitute the algorithm, that is, by changing the execution sequence. Updating the algorithm includes modifying or excluding one or more of the sub-modules that constitute the algorithm, or adding one or more sub-modules other than the sub-modules that constitute the algorithm. In addition, updating the algorithm includes changing the parameters.

Meanwhile, when the analysis algorithm is updated, in this specification, the analysis algorithm that is the target of the update is referred to as a "legacy analysis algorithm" and the analysis algorithm that is the result of the update is referred to as an "update analysis algorithm."

In addition, the result of applying the fluorescence data to the legacy analysis algorithm is referred to as the "legacy analysis data," and the result of applying the fluorescence data to the update analysis algorithm is referred to as the "update analysis data."

The term "metadata" refers to data that describes the fluorescence data according to certain rules. The metadata may include information about the reaction vessel, reagent, or signal generation means described above. In addition, the metadata may include information about the device used to generate the fluorescence data.

Hereinafter, various implementation examples of the present disclosure will be described with reference to the drawings.

FIG. 1 exemplarily illustrates, according to one embodiment, a detection device 2000 and a display device (1000, hereinafter referred to as display device) for displaying a performance comparison result for a fluorescence data analysis algorithm connected to the detection device 2000. However, FIG. 1 is only an example. The display device 1000 and the detection device 2000 may be connected to each other by wired or wireless communication. However, the block diagram illustrated in FIG. 1 is only an example, and the spirit of the present disclosure is not to be construed as being limited to what is illustrated in FIG. 1. For example, components not illustrated in FIG. 1 may be additionally connected to the display device 1000 and the detection device 2000. Alternatively, unlike what is illustrated, the display device 1000 may be implemented by being included in the detection device 2000. However, the following description will assume that the above-described components 1000 and 2000 are implemented or connected as illustrated in FIG. 1. Hereinafter, respective components will be described in detail.

The detection device 2000 is implemented to perform a nucleic acid amplification reaction and a nucleic acid detection operation on a sample. According to one embodiment, the detection device 2000 may be implemented to perform the nucleic acid detection operation without performing the nucleic acid amplification reaction, but the following description will assume that the detection device 2000 is implemented to perform the nucleic acid amplification reaction as well.

Meanwhile, as the above-described nucleic acid amplification reaction is performed, when a sample in a reaction vessel includes a target analyte, the amount of target analyte is not only amplified, but a magnitude of the signal generated by the above-described signal generation means may also be amplified as described above. Accordingly, the detection device 2000 is implemented to detect the magnitude of the signal. Specifically, the detection device 2000 may monitor in real time the magnitude of the above-described signal that changes as the nucleic acid amplification reaction progresses. The monitored result may be output from the detection device 2000 in the form of a data set as mentioned in the definition of the term, for example, as signal intensity per cycle. Here, the signal intensity per cycle is information as a material that serves as a basis for determining the presence or absence of the target analyte in the corresponding sample.

Meanwhile, since the magnitude of the signal generated by the signal generation means may also be amplified when the above-described nucleic acid amplification reaction is performed, the nucleic acid amplification reaction will be considered below as causing the signal generation reaction, which is previously described as a term.

As described above, in the amplification reaction of the target analyte, the signal may be detected by the signal generation means, and the detected signal may include fluorescence data. The detected signal may undergo a data processing process configured by various algorithms.

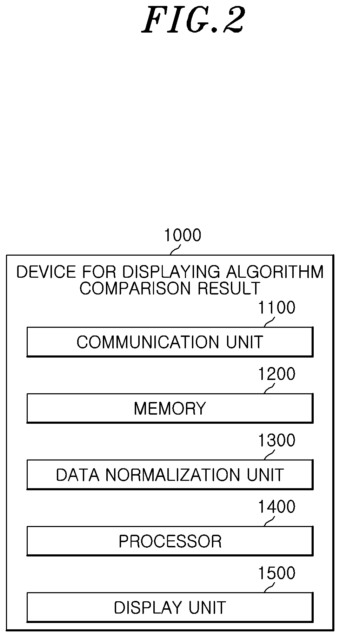

Hereinafter, each functional component of the display device 1000 will be described in more detail with reference to FIG. 2.

FIG. 2 conceptually illustrates the configuration of the display device 1000 according to one implementation example. Before referring to FIG. 2, it should be noted that the display device 1000 may be implemented in a laptop, a server, or a cloud, but is not limited thereto.

The display device according to one implementation example includes a memory 1200 that stores at least one command, fluorescence data for an amplification reaction of a target analyte, and legacy analysis data for the target analyte which is a result of applying a legacy analysis algorithm to the fluorescence data, a display unit 1500, and a processor 1400, in which the at least one command is executed by the processor to obtain update analysis data for the target analyte, which is a result of applying an update analysis algorithm of the legacy analysis algorithm to the fluorescence data, and display a comparison result of the legacy analysis data and the update analysis data is displayed on the display unit 1500.

Referring to FIG. 2, the display device 1000 according to an implementation example includes a communication unit 1100, the memory 1200, a data normalization unit 1300, the processor 1400, and the display unit 1500, but is not limited thereto.

First, the communication unit 1100 is implemented as a wired or wireless communication module. Through the communication unit 1100, the display device 1000 may communicate with the outside based on the algorithm comparison result. For example, as will be described later, various types of data stored in the memory may be data received by the display device 1000 from the outside through the communication unit 1100. Here, the 'outside' may be the above-described detection device 2000 or a preparation device used for preparing molecular diagnosis, or may be a software module used for driving each of these devices.

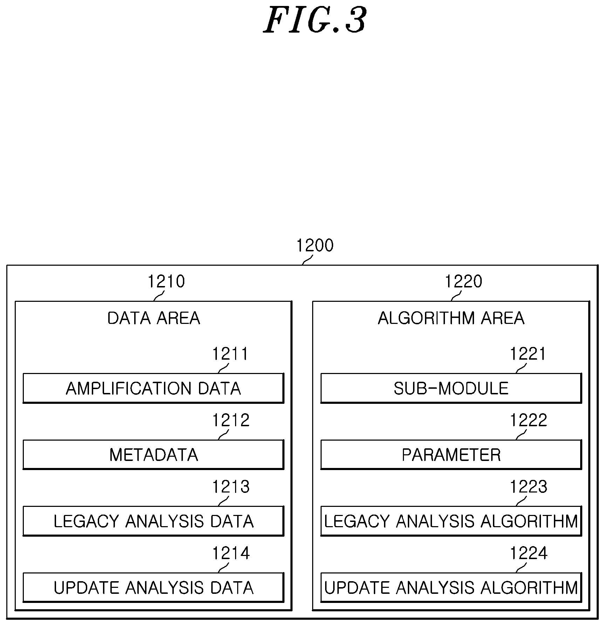

Next, the memory 1200 will be described. Various types of information are stored in the memory 1200. For example, various types of data, predefined analysis algorithms, and their components may be stored. The information stored in the memory 1200 will be described below with reference to FIG. 3.

Meanwhile, in FIG. 2, the memory 1200 is illustrated as a separate component from the processor 1400, but the memory 1200 may be implemented as a single device with the processor 1400. For example, the memory 1200 may be a storage such as a cache included inside the processor 1400.

Next, the data normalization unit 1300 will be described. In order for the data stored in the memory to be applied to the analysis algorithm, the format of the data should be a format that can be applied to the analysis algorithm. In this case, when the format of the data is a format that cannot be applied to the analysis algorithm, the format of the data may be modified to a predefined data standard format through the data normalization unit 1300. The data standard format may be different for each analysis algorithm, and the data normalization unit 1300 can modify the data format according to the analysis algorithm.

Next, the processor 1400 can be implemented by a central processing unit (CPU), a graphics processing unit (GPU), a micro controller unit (MCU), or a dedicated processor on which the methods according to one embodiment are performed, etc. In addition, the processor 1400 may be implemented by at least one core.

The processor 1400 may write data to the memory 1200. In addition, the processor 1400 may read out and execute commands stored in the memory 1200. For example, the processor 1400 may cause the display device 1000 to perform functions to be described below by executing the commands stored in the memory 1200.

Meanwhile, the display unit 1500 of the display device 1000 can be implemented by a means for displaying or receiving data. For example, the display unit 1500 may be implemented through a touch screen, a touch pad, an LCD monitor, etc. However, the present disclosure is not limited thereto.

Hereinafter, information that can be stored in the memory will be described in more detail with reference to FIG. 3.

FIG. 3 illustrates a type of information stored in the memory.

Refer to FIG. 3. Various types of information are stored in the memory 1200. The memory 1200 includes a data area 1210 and an algorithm area 1220, and information related to data and information related to an algorithm may be stored, respectively. However, the present disclosure is not limited thereto. For example, commands (not illustrated) executable by the processor 1400 may also be stored in the memory.

First, the data area 1210 of the memory 1200 will be described. The data area 1210 may store fluorescence data 1211, metadata 1212, legacy analysis data 1213, and update analysis data 1214. However, the present disclosure is not limited thereto.

As described above, the fluorescence data 1211 is obtained through the amplification reaction and is data that includes an amplification cycle. In this case, the fluorescence data may be obtained from a nucleic acid amplification reaction.

The metadata 1212 is data that describes the fluorescence data 1211, and the metadata may include information about the amplification reaction from which the fluorescence data has been obtained, such as a reaction vessel, a reagent, or a signal generation means. In addition, it may include at least one of information about a target analyte, information about the reaction vessel used for the reaction to obtain the fluorescence data, information about the detection reagent used for detecting the target analyte, information about consumables, or information about equipment.

The metadata 1212 will be described in more detail. For example, the memory 1200 may store the fluorescence data in units of plates or strips. In addition, the memory 1200 may store data for multiple plates and the fluorescence data generated based on the data for the multiple plates. Here, the data for the plate includes numbers or letters for identifying the plate, information recorded for the plate, or data for reaction wells included in the plate. Among these, the information recorded for the plate includes various types of information such as the date, time, and method of performing the amplification reaction on the plate. In addition, the data for the reaction wells include data sets for each of the reaction wells, noise-removed data sets, amplification curves, Ct values, signal values, etc. In this case, the metadata 1212 may include various types of information included in the fluorescence data. In addition, when multiple sets of fluorescence data are stored in the memory, the memory may store metadata for each of the multiple sets of fluorescence data.

Such metadata may be utilized for searching the fluorescence data, so the fluorescence data applied to the analysis algorithm may be used to be easily searched. The multiple fluorescence data 1211 and the legacy analysis data 1213 corresponding to each of the multiple the fluorescence data are stored in the memory 1200 of the display device 1000 according to one implementation example, and at least one command stored in the memory is executed by the processor, so search conditions may be input, the fluorescence data may be searched using the search conditions among the multiple sets of fluorescence data, and the obtained update analysis data may be a result of the update analysis algorithm being applied to the searched fluorescence data.

In this case, data that has been previously input and stored may be present in the memory in advance. Such data may be data regarding multiple types of target analytes, and only data regarding a specific target analyte among the target analytes may be found through the search.

In this case, the memory may store the metadata for each of the multiple sets of fluorescence data, and the search conditions may include the metadata.

Before describing the legacy analysis data 1213 and the update analysis data 1214, the algorithm area 1220 will be described first.

The algorithm area 1220 may store a sub-module 1221, a parameter 1222, a legacy analysis algorithm 1223, and an update analysis algorithm 1224. However, the present disclosure is not limited thereto.

As described above, at least one sub-module 1221 including the parameter 1222 may be configured to combine the analysis algorithm. In this case, the type and arrangement order of at least one sub-module constituting the analysis algorithm are called an execution sequence. The analysis algorithm may be updated by changing the execution sequence. In this case, the analysis algorithm that is being updated, that is, the target of the update, is the legacy analysis algorithm 1223, and the analysis algorithm that the legacy analysis algorithm is updated is the update analysis algorithm 1224. That is, the analysis algorithm and its components before and after the update may be stored in the algorithm area 1220.

Describing the legacy analysis data 1213 and the update analysis data 1214 again, the result data of the fluorescence data 1211 applied to the legacy analysis algorithm 1223 is the legacy analysis data 1213, and the result data of the fluorescence data 1211 applied to the update analysis algorithm 1224 is the update analysis data 1214.

Meanwhile, the data stored in the memory 1200 may be normalized by the data normalization unit 1300 that may be included in the display device 1000 according to one implementation example. As described above, the fluorescence data and the legacy analysis data stored in the memory may be multiple sets, and data of each of the multiple sets may be normalized by the data normalization unit 1300.

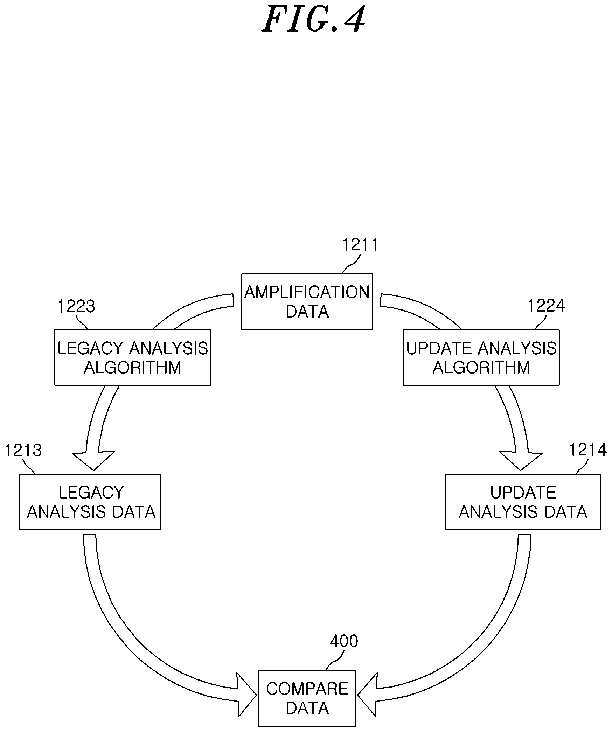

Hereinafter, the configuration in which the multiple algorithms are applied to the fluorescence data will be examined in more detail with reference to FIG. 4.

FIG. 4 conceptually illustrates a configuration in which each result data generated by applying multiple algorithms to fluorescence data is compared with each other according to one implementation example.

Refer back to FIG. 4. The fluorescence data 1211 may be applied to the legacy analysis algorithm 1223 and the update analysis algorithm 1224. In this case, the fluorescence data 1211 may be generated from, for example, the detection device 2000. The legacy analysis algorithm 1223 means the 'existing' algorithm before being updated. Through at least one process of changing or removing the sub-module constituting the legacy analysis algorithm and changing the execution sequence, or adding a new sub-module, the legacy analysis algorithm may be updated to the update analysis algorithm. In this way, two analysis algorithms that have differences through the update can be compared with each other using result data obtained by applying the same data. Referring to FIG. 4, it can be confirmed that the same data, i.e., the fluorescence data 1211 is input to the legacy analysis algorithm 1223 and the update analysis algorithm 1224, and a comparison 400 of the result data is subsequently performed.

Meanwhile, as described above, the multiple sets of fluorescence data 1211 may be stored in the memory 1200, and when the multiple sets of fluorescence data are applied to the legacy analysis algorithm or the update analysis algorithm, the multiple sets of fluorescence data may be applied in parallel to data in units of reaction vessels. In this case, the data in units of reaction vessels may be included in the metadata described above. The reaction vessel may be a well, a well strip, or a plate.

Hereinafter, the update of this algorithm will be described in more detail.

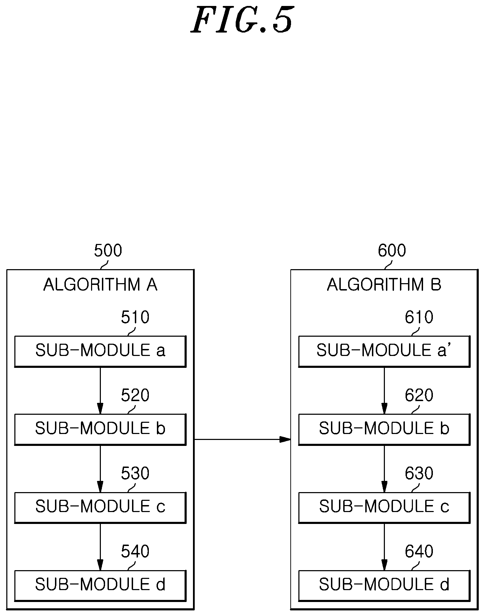

FIG. 5 conceptually illustrates a configuration in which an algorithm is updated according to one implementation example. However, FIG. 5 is only an example.

Refer to FIG. 5. Algorithm A(500) and algorithm B(600) may each include the sub-module. The sub-module is a predetermined unit of a signal processing process that analyzes or processes a signal value as described above. More specifically, the sub-module may be a part of a program that is included in the algorithm and is organically operated for the purpose of the algorithm. For example, it is assumed that the algorithm A is the DSP described above. In this case, the sub-module may be each step that constitutes the DSP. That is, each step such as a step of correcting the data set, a step of generating the non-linear function for the corrected data set to determine the fitting accuracy of the non-linear function for the corrected data set, and a step of determining the presence or absence of the target analyte in the sample using the fitting accuracy may be the sub-module.

The legacy analysis algorithm and the update analysis algorithm according to one implementation example may include at least one of the sub-module used for processing the fluorescence data or the sub-module used for determining the presence or absence of the target analyte in the sample.

Meanwhile, the algorithm may need to be updated depending on the development environment, and the update of the algorithm may be performed by modifying the sub-module. Referring to FIG. 5, the algorithm A may be updated by changing or removing some of the included sub-modules, changing the execution sequence which is the operation order of the sub-module, or adding a new sub-module.

Referring to FIG. 5, it may be confirmed that a sub-module a(510) is changed to a sub-module a'(610), an order of a sub-module b(520) and a sub-module c(530) is replaced, a sub-module d(540) is removed, and a new sub-module d(640) is added, while the algorithm A is updated to the algorithm B. However, FIG. 5 is only an example, and the update may be performed by applying only some of changing or removing of the sub-module or adding a new sub-module.

Hereinafter, the algorithm that may be applied to the fluorescence data will be described in more detail, using the above-described DSP as an example.

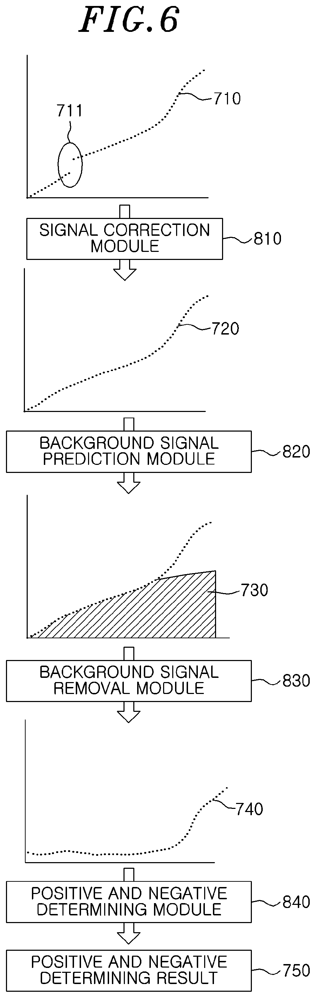

FIG. 6 conceptually illustrates a configuration in which fluorescence data generates positive and negative determining results of a target analyte through a plurality of sub-modules of a DSP algorithm according to an implementation example. However, FIG. 6 is only an example.

Referring to FIG. 6, an amplified signal 710 may be applied to a signal correction module 810. The amplified signal 710 may be a signal that is generated in the detection device 2000 and then is not applied to a separate signal correction algorithm. The signal correction module 810 may be an algorithm that corrects noise. For example, referring to FIG. 6, it may be confirmed that the amplified signal 710 includes noise 711 that causes discontinuity in a signal waveform. When the signal processing is performed without correcting this discontinuous signal, incorrect determining may occur later. Therefore, the correction of noise that may affect the determining result is required, and the noise correction may be performed through the signal correction module 810.

It is assumed that the signal, which has passed through the above-described signal correction module, is a correction signal 720. Referring to the correction signal 720 of FIG. 6, it may be confirmed that the noise 711 in the amplified signal 710 has been corrected.

The correction signal 720 may be separated into a background signal 730 and other signals through a background signal prediction module 820. Here, the background signal 730 means a signal of the reaction sample itself and a signal of the measurement system itself, not the signal generated by the amplification reaction. For example, in the case of the PCR reaction, in a baseline region, which is a region where there is little change in the fluorescence signal during an initial cycle of the PCR, the fluorescence signal emitted by a PCR reaction product is not sufficient to be detected, so the background signal, which is the fluorescence signal of the reaction sample itself and the fluorescence signal of the measurement system itself, may occupy most of the fluorescence signal in the baseline region.

When the correction signal 720 passes through a background signal removal module 830, a positive and negative determining signal 740 with the background signal 730 removed may be generated. The generated positive and negative determining signal 740 may be applied to a positive and negative determining module 840, so positive and negative determining results 750 may be generated.

Hereinafter, one embodiment of a performance comparison result display for the fluorescence data analysis algorithm will be described.

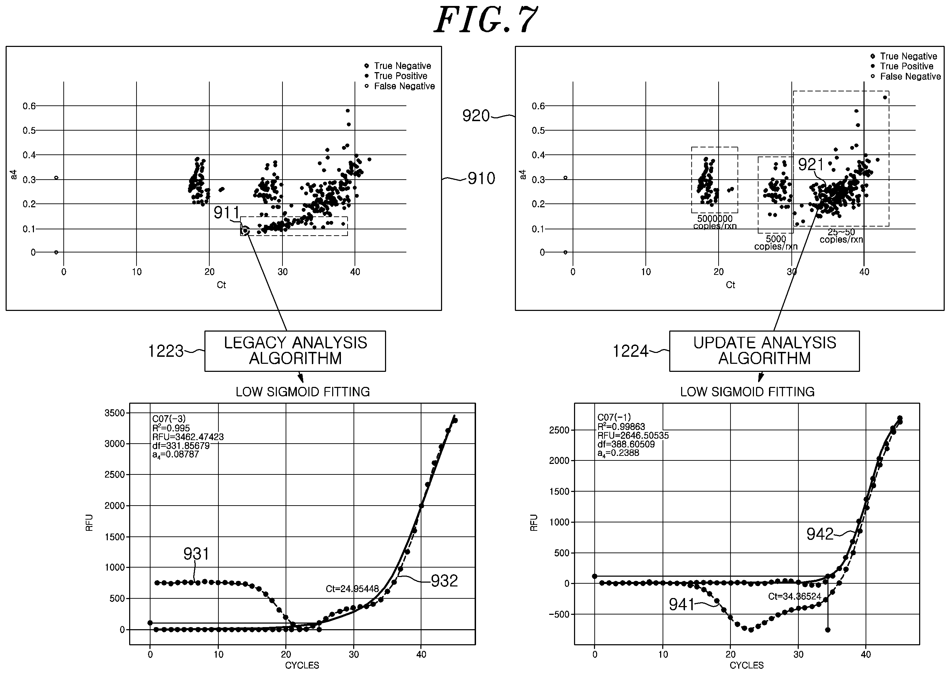

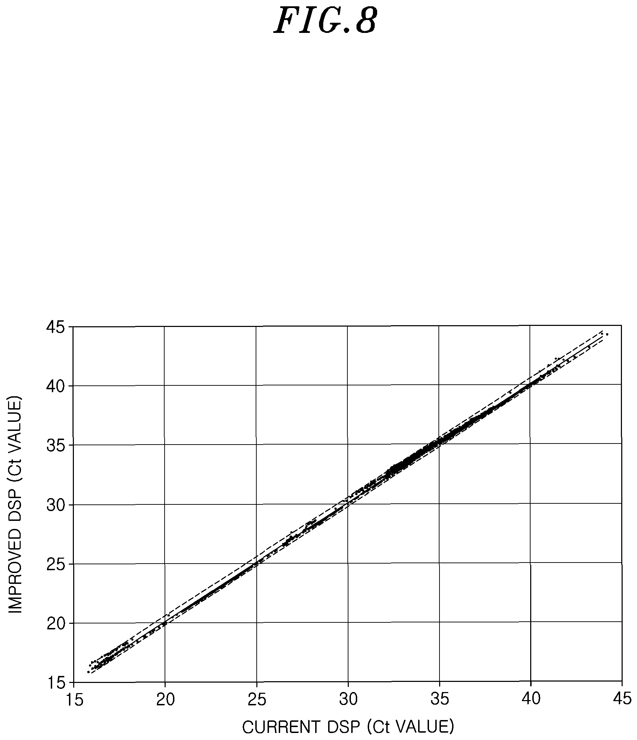

In FIGS. 7 and 8, the algorithm result data is illustrated in a parameter plot according to one implementation example.

FIG. 7 illustrates analysis data for the legacy analysis algorithm and the update analysis algorithm, plotted on a Ct - α4 plane, according to an implementation example. However, FIG. 7 is only an example.

In the display unit of the update comparison result display device according to an implementation example, the determination comparison result for the presence or absence of the target analyte for the legacy analysis data and the update analysis data may be displayed, or the Ct value comparison distribution may be displayed.

Referring to FIG. 7, a plot 910 applied to the legacy analysis algorithm 1223 and a plot 920 applied to the update analysis algorithm 1224 are illustrated for the same fluorescence data. First, the difference between the two algorithms will be described. The legacy analysis algorithm performs a predetermined data analysis on the input data to output the result data. The update analysis algorithm, in which at least a part of the legacy analysis algorithm is modified, receives the same data 931 and 941 applied to the legacy analysis algorithm and outputs result data 932 and 942 for the data 931 and 941. Referring to FIG. 7, the two result data are a kind of amplification curves, and it may be confirmed that the baseline is formed differently and the analysis on the determining is different.

Specifically, when the corresponding data are respectively plotted on a plane defined by α4, a parameter related to amplification efficiency, and cycle threshold (Ct), it can be observed that the positions of the data are different (911, 921). In addition, it can be confirmed that the distribution pattern of the data in the area where the positive and negative determination is ambiguous in the legacy analysis algorithm has been changed in the result data for the update analysis algorithm. In addition, it can be confirmed that the result data of the update analysis algorithm exhibit a higher degree of clustering by target concentration than the result data of the legacy analysis algorithm (clustering for each target concentration is not illustrated in the result data of the legacy analysis algorithm). Through these changes, a user may confirm whether the update of the algorithm has been completed.

FIG. 8 illustrates Ct values for each of the legacy analysis algorithm and the update analysis algorithm, plotted on one plane, according to one implementation example. However, FIG. 8 is only an example.

FIG. 8 illustrates two result data as a data set on one plane. Referring to FIG. 8, the result data is displayed on a plane where a horizontal axis is the Ct value obtained from the result data of the legacy analysis algorithm and a vertical axis is the Ct value obtained from the result data of the update analysis algorithm. In this case, when the Ct values of the result data of the legacy analysis algorithm and the result data of the update analysis algorithm are the same, the data set consisting of these Ct values will be displayed on a y=x straight line. In other words, it may be confirmed that the farther the data set displayed as a point on the plane deviates from the y=x straight line, the greater the difference in the Ct values between the two algorithms. This analysis method may be effectively used when confirming the distribution of the difference in the Ct values.

The applied legacy analysis data on the update comparison result display device according to one implementation example is written for the purpose of verifying the performance of the diagnostic reagent of the target analyte for submitting a designated certification authority, and when the comparison result of the legacy analysis data and the update analysis data fall within a predetermined range, the update from the legacy analysis algorithm to the update analysis algorithm may be executed.

In addition, the legacy analysis data applied on the update comparison result display device according to one implementation example may be written for the purpose of verifying the performance of the diagnostic reagent of the target analyte for submitting to the designated certification authority.

That is, by using the plot analysis as illustrated in FIG. 8, it is possible to review whether to apply the update by comparing the data submitted to the designated certification authority with the update analysis data.

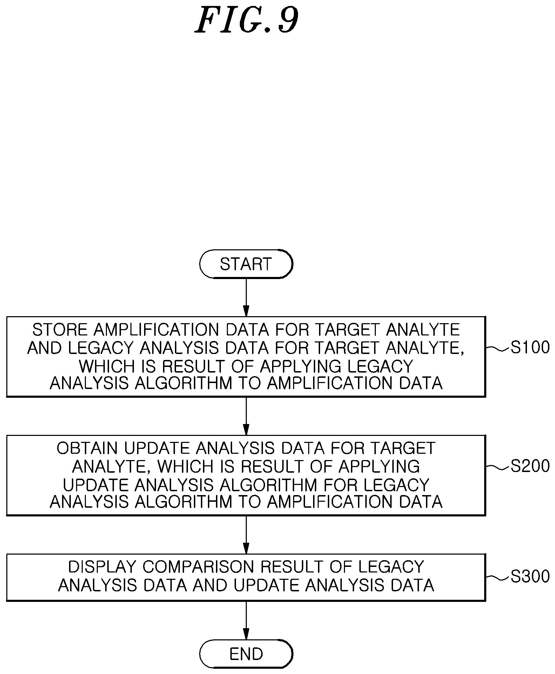

FIG. 9 is a flowchart of a method for displaying an algorithm comparison result according to one implementation example. However, FIG. 9 is only an example.

The method for displaying an algorithm comparison result according to one implementation example is a method for comparing and analyzing multiple algorithms for analyzing fluorescence data for an amplification reaction of a target analyte, in which the method is performed by a computer device and includes: storing fluorescence data for the target analyte and legacy analysis data for the target analyte, which is a result of applying a legacy analysis algorithm to the fluorescence data (S100); obtaining update analysis data for the target analyte, which is a result of applying an update analysis algorithm of the legacy analysis algorithm to the fluorescence data (S200); and displaying a comparison result of the legacy analysis data and the update analysis data (S300).

The method for comparing and analyzing algorithms according to one implementation example is performed by a computer device, and includes: receiving the amplification data and a legacy analysis algorithm, the amplification data including i) metadata for the nucleic acid amplification reaction and ii) fluorescence data indicating an amplification result of the nucleic acid amplification reaction; generating normalized data using data extracted and converted from the input amplification data; storing i) the normalized data and ii) legacy analysis data which is the result of applying the legacy analysis algorithm to the normalized data in a normalized database (DB) pre-built in a memory of the computer device, the normalized DB storing, for the target analyte, a plurality of normalized data and the legacy analysis data for the plurality of normalized data; receiving a search condition including the metadata and/or the fluorescence data; searching for normalized data meeting the search condition among the plurality of normalized data stored in the normalized DB and the legacy analysis data for the normalized data; receiving an update analysis algorithm of the legacy analysis algorithm; comparing the update analysis data, which is a result of applying the update analysis algorithm to the searched normalized data, with the searched legacy analysis data; and displaying the comparison result.

The normalized data may be generated by converting data extracted from the metadata so that the received metadata conforms to a predetermined format. That is, the input data may undergo extraction and conversion to become the normalized data, and the data that has passed this process may be loaded into the normalized data base.

Meanwhile, the metadata included in the normalized data may be configured as a hierarchy that is a parent-child relationship between data. For example, it is assumed that the metadata includes project information, product information, and reaction vessel information. For example, the project information may include reagent development project information, the product information may include equipment, consumables, and product information used in a diagnostic reagent product developed in a reagent development project, oligo composition information of the diagnostic reagent product developed in the reagent development project, and sample information used during development in the reagent development project, and the reaction vessel information may include composition information such as plates and wells of the diagnostic reagent product. In this case, the parent-child relationship may be established between the project information, the product information, and the reaction vessel information. For example, the project information may have multiple pieces of product information as its child, and the product information may have multiple pieces of reaction vessel information as its child. In this case, it may be considered that the information is structured hierarchically from top to bottom in the order of the project information, the product information, and the reaction vessel information. In this way, the metadata included in the normalized data may have the hierarchy.

## Claims

1. A computer device, comprising:
a memory that stores at least one instruction, fluorescence data for an amplification reaction of a target analyte, and legacy analysis data for the target analyte, the legacy analysis data being a result of applying a legacy analysis algorithm to the fluorescence data;
a display unit; and
a processor,
wherein the at least one instruction is executed by the processor
to obtain update analysis data for the target analyte, which is a result of applying an update analysis algorithm of the legacy analysis algorithm to the fluorescence data, and
to display a comparison result of the legacy analysis data and the update analysis data on the display unit.

2. The computer device of claim 1, wherein the fluorescence data is a fluorescence signal detected in a reaction vessel where a nucleic acid amplification reaction occurs.

3. The computer device of claim 1, wherein the legacy analysis algorithm and the update analysis algorithm include at least one of a sub-module used for processing the fluorescence data and a sub-module used for determining the presence or absence of the target analyte in a sample.

4. The computer device of claim 1, wherein the update analysis algorithm is generated by modifying or excluding one or more of sub-modules in the legacy analysis algorithm, or generated by adding one or more sub-modules other than the sub-modules in the legacy analysis algorithm, or generated by changing an execution sequence of one or more of the sub-modules in the legacy analysis algorithm.

5. The computer device of claim 1, wherein the memory stores a plurality of sets of fluorescence data and legacy analysis data for the plurality of sets of fluorescence data,
the at least one instruction is executed by the processor to input a search condition and then search for the fluorescence data using the search condition among the plurality of sets of fluorescence data, and
the obtained update analysis data is a result of applying the update analysis algorithm to the searched fluorescence data.

6. The computer device of claim 5, wherein the memory stores metadata for each of the plurality of sets of fluorescence data, and
wherien the search condition includes metadata.

7. The computer device of claim 6, wherein the metadata includes at least one one or more selected from the group consisting of information generated during a development process of a reagent for detecting the target analyte, information on the reagent, information on the target analyte, identification (ID) information on a sample used in the nucleic acid amplification reaction, identification (ID) information on a reaction vessel used in a reaction to obtain amplification data, temperature information, enzyme master mix information, equipment information, and consumables information.

8. The computer device of claim 7, wherein the plurality of sets of fluorescence data are obtained in respective reaction vessels and are applied to either the legacy analysis algorithm or the update analysis algorithm.

9. The computer device of claim 8, wherein the reaction vessel is a well, a well strip, or a plate.

10. The computer device of claim 1, wherein the legacy analysis data is data which have been prepared to verify performance of a diagnostic reagent of the target analyte for submitting to a predetermined certification authority, and
when the comparison result of the legacy analysis data and the update analysis data falls within a predetermined range, an update from the legacy analysis algorithm to the update analysis algorithm is executed.

11. The computer device of claim 1, further comprising:
a data normalization unit,
wherein the memory stores the fluorescence data and the legacy analysis data as a plurality of data sets, and the plurality of sets of fluorescence data and legacy analysis data are standardized by the data normalization unit.

12. The computer device of claim 1, wherein the display unit displays a determination comparison result of the presence or absence of the target analyte for the legacy analysis data and the update analysis data, or displays a Ct value comparison distribution of the target analyte for the legacy analysis data and the update analysis data.

13. The computer device of claim 1, wherein the legacy analysis data is prepared to verify performance of a diagnostic reagent of the target analyte, and submitted to a predetermined certification authority.

14. A method for comparing and analyzing a plurality of algorithms for analyzing fluorescence data for an amplification reaction of a target analyte, the method being performed by a computer device comprising:
storing fluorescence data for the target analyte and legacy analysis data for the target analyte, which is a result of applying a legacy analysis algorithm to the fluorescence data;
obtaining update analysis data for the target analyte, which is a result of applying an update analysis algorithm of the legacy analysis algorithm to the fluorescence data; and
displaying a comparison result of the legacy analysis data and the update analysis data.

15. A method for comparing and analyzing a plurality of algorithms for analyzing amplification data obtained from a nucleic acid amplification reaction of the target analyte, the method being performed by a computer device comprising:
receiving the amplification data and a legacy analysis algorithm, the amplification data including i) metadata for the nucleic acid amplification reaction and ii) fluorescence data indicating an amplification result of the nucleic acid amplification reaction;
generating normalized data using data extracted and converted from the input amplification data;
storing i) the normalized data and ii) legacy analysis data which is a result of applying the legacy analysis algorithm to the normalized data in a normalized database (DB) pre-built in a memory of the computer device, the normalized DB storing, for the target analyte, a plurality of normalized data and legacy analysis data for the plurality of normalized data;
receiving a search condition including the metadata and/or the fluorescence data;
searching for normalized data meeting the search condition among the plurality of normalized data stored in the normalized DB and legacy analysis data for the normalized data;
receiving an update analysis algorithm of the legacy analysis algorithm;
comparing update analysis data, which is a result of applying the update analysis algorithm to the searched normalized data, with the searched legacy analysis data; and
displaying a comparison result.

16. The method of claim 15, wherein the metadata includes one or more selected from the group consisting of information generated during a development process of a reagent for detecting the target analyte, information on the reagent, information on the target analyte, identification (ID) information on a sample used in the nucleic acid amplification reaction, identification (ID) information on a reaction vessel used in a reaction to obtain the amplification data, temperature information, enzyme master mix information, equipment information, and consumables information.

17. The method of claim 15, wherein the fluorescence data is a fluorescence signal detected in a reaction vessel where the nucleic acid amplification reaction occurs.

18. The method of claim 15, wherein the normalized data is generated by converting data extracted from the metadata so that the received metadata conforms to a predetermined format.

19. The method of claim 15, wherein the metadata included in the normalized data is structured in a hierarchy representing a parent-child relationship among data.

20. A computer program stored on a computer-readable recording medium programmed to perform, each step included in the method of one of claims 14 to 19.

21. A computer-readable recording medium storing a computer program programmed to perform, each step included in the method of one of claims 14 to 19.
